# EUROPEAN PATENT APPLICATION

(11) **EP 3 369 804 A1**
(43) Date of publication of application: **05.09.2018**
(21) Application number: 16859763.1
(22) Date of filing: 25.10.2016
(51) Int. Cl.: C12M 1/26, C12N 1/02, C07K 14/765, C12N 1/00, C12N 5/07

(54) **CELL-SPREADING METHOD AND CELL-SPREADING KIT FOR OBSERVING RARE CELLS**

(30) Priority: 26.10.2015 JP 2015209744
(71) Applicant: Konica Minolta, Inc., Tokyo 100-7015 (JP)
(72) Inventor: ARAKI, Jungo, Tokyo 100-7015 (JP); MASUBUCHI, Manami, Tokyo 100-7015 (JP)
(74) Representative: Hoffmann Eitle
(86) International application number: PCT/JP2016/081512
(87) International publication number: WO 2017/073533

(57) **Abstract**

An object of the present invention is to provide a means that enables improvement of efficiency in recovery of rare cells in a cell suspension by suppressing cell adsorption to laboratory equipment but not suppressing cell adsorption to a cell spreading substrate. A cell spreading method according to the invention includes a step of adding to a cell suspension a protein in such an amount as to give a final concentration of from 0.0001% to 0.1%, and preferably from 0.0005% to 0.01% (a protein addition step) and a step of spreading cells contained in the cell suspension that has been subjected to the protein addition step on a cell spreading substrate having a contact angle with water of 60 degrees or more, and preferably from 70 to 90 degrees, and allowing to stand to cause the cells to adsorb onto the substrate (a cell spreading step).

## Description

### Technological Field

The present invention relates to a cell spreading method and a cell spreading kit for performing the method.

### Description of the Related Art

Blood usually contains blood cells such as erythrocytes and leukocytes (neutrophils, eosinophils, basophils, lymphocytes, and monocytes), and may also contain rare cells such as circulating tumor cells (CTCs), circulating endothelial cells (CECs), circulating endothelial progenitors (CEPs), and other precursor cells. Additionally, cultured cell populations may contain stem cells, specific differentiated cells, and other characteristic cells.

For example, blood circulating tumor cells (CTCs) are cells found in the blood of patients with breast cancer, lung cancer, prostate cancer, pancreas cancer, and the like, and are drawing attention by being clinically important information, such as that the number of CTCs reflects cancer metastasis. However, CTC density in the blood is extremely low (low-density cases contain approximately from one to 10 cells per 10 mL of whole blood), and therefore detection and counting of CTCs are not easy. In addition, profiling of biomarkers expressed in CTCs is important for advancing research on CTCs from various viewpoints, such as elucidation of a metastatic mechanism through a comparison with profiling of biomarkers of the primary cancer, determination of effective anticancer drugs (molecular targeted drugs), and specification of CTC subpopulations (CTCs having characteristics of epithelial cells, mesenchymal cells, stems cells, and the like).

When detecting rare cells such as CTCs or characteristic cells for the purposes as described above, it is desirable to individually determine shapes of the cells, states of staining, and the like by microscopic observation and the like. There are known methods of detecting CTCs in blood by performing nuclei staining, fluorescent staining, and the like to specify positions of cells. For example, as described in Patent Document 1 (WO 2014/007191), a system is used that spreads cells contained in a cell suspension in such a manner that microscopic observation and staining are facilitated by using a cell spreading device produced by providing a flow channel forming frame on a substrate having a large number of microchambers (well structures) capable of accommodating cells on a surface thereof. In this system, the cell suspension is introduced in a flow channel of the cell spreading device, the cells are recovered into the microchambers while causing the cell suspension to move through the flow channel, and then the cell suspension is discharged from the flow channel. Additionally, there is also a method of spreading cells by using a cell spreading substrate adapted to easily complement cells by providing a structure other than the microchambers, like grooves, or a method of spreading cells by using a cell spreading substrate not having such a structure.

The present inventors found that, in the system as described above, by adding to a cell suspension containing PBS as solvent a small amount of protein so as to give a final concentration in a specific range, and next, spreading the cell suspension on a cell spreading substrate having a contact angle with water in a specific range, cells contained in the cell suspension can be efficiently recovered in microchambers provided on the cell spreading substrate while suppressing nonspecific adsorption of the cells to laboratory equipment used for preparation and spreading of the cell suspension, and thereby completed the present invention. It is surprising that the above two effects can be both achieved only by controlling the concentration of the protein that is added to the cell suspension.

### Citation List

Patent Document

[Patent Document 1]: WO 2014/007191

### Summary

### Technological Problem

Since rare cells such as CTCs are present in very small numbers, adsorption of the rare cells to surfaces of pieces of laboratory equipment, such as a pipette tip and a micro tube, that are used for preparation of a cell suspension, introduction of the cell suspension to the cell spreading device of the aforementioned system, and the like can have influence on counting accuracy for the number of the cells.

On the other hand, the system using the cell spreading substrate (device) as described in Patent Document 1 requires adsorption of the rare cells or the like to bottom surfaces of the microchambers. In order to count the number of the rare cells, the shapes of cells recovered in the microchambers, states of staining, and the like may be checked one by one for determination. However, since a plurality of cells is sometimes recovered in one microchamber, it is desirable to cause all the cells to adsorb to the bottom surfaces of the microchambers so that the cells do not overlap one another. Additionally, when rare cells or the like are caused to adsorb to the bottom surfaces of the microchambers, it can be prevented that, along with movement of the cell suspension, the rare cells or the like once recovered are lost by flowing out again from the microchambers.

Patent Document 1 describes a method for producing microchambers, in which, in order to suppress nonspecific adsorption of the cells to a surface other than the microchambers of the cell spreading substrate so as to ensure that the cells can be recovered in the microchambers, a blocking treatment liquid containing a blocking agent (e.g., bovine serum albumin (BSA), a hydrophilic polymer, and/or a phospholipid) is contacted with an upper surface of the cell spreading substrate in a predetermined manner to coat the surface other than the microchambers or the surface other than the microchambers and inner wall surfaces of the microchambers with the blocking agent, while keeping the bottom surfaces of the microchambers uncoated with the blocking agent so that the cells can adsorb thereto.

However, Patent Document 1 does not particularly describe suppression of adsorption of the rare cells to surfaces of laboratory equipment used before spreading the cells on the cell spreading substrate. As described in Patent Document 1, it is conventionally general that PBS is used as a solvent of a cell suspension containing cells (which may be previously subjected to an immobilization treatment), and a hydrophobic material (for example, polystylene subjected to a hydrophilic treatment as needed and having a contact angle with water of from 20 to 100 degrees) is used for a cell spreading substrate onto which the cell suspension is to be dropped. However, under the present conditions, there occurs a problem where the cells adhere nonspecifically to the laboratory equipment, such as a pipette tip and a micro tube, needed for preparation of the cell suspension or introduction of the cells to the cell spreading substrate described above.

It is an object of the present invention to provide a means that enables improvement of efficiency in recovery of rare cells in a cell suspension by suppressing cell adsorption to laboratory equipment but not suppressing cell adsorption to a cell spreading substrate.

### Solution to Problem

In one aspect, the present invention provides a cell spreading method including a step of adding to a cell suspension a protein in such an amount as to give a final concentration of from 0.0001% to 0.1% (a protein addition step) and a step of spreading cells contained in the cell suspension that has been subjected to the protein addition step on a cell spreading substrate having a contact angle with water of 60 degrees or more, and allowing to stand to cause the cells to adsorb onto the cell spreading substrate (a cell spreading step).

Additionally, in a further aspect, the invention provides a cell spreading kit including (a) a cell dilution containing a protein with a concentration of from 0.0001 % to 0.1% or the protein and a solvent for preparing the cell dilution and (b) a cell spreading substrate having a contact angle with water of 60 degrees or more.

### Advantageous Effects of Invention

Use of the cell spreading method of the present invention greatly reduces loss of rare cells in a cell suspension, thereby enabling more cells to be recovered on a cell spreading substrate (in microchambers) than in conventional methods. Additionally, also during an assay after cell spreading, cells can be retained while adsorbing to the cell spreading substrate (bottom surfaces of the microchambers) with sufficient adsorption strength, and even when a staining solution or the like is introduced into the flow channel, the cells do not come off and stay on the surface of the cell spreading substrate (bottoms of the microchambers), so that another step of detecting the rare cells can be performed subsequent to the cell spreading step.

### Brief Description of the Drawing

Fig. 1 is a flowchart depicting an embodiment of a cell spreading method of the present invention,
Fig. 2 is a schematic diagram illustrating an embodiment of a cell detection system (2A) and a cell spreading substrate (2B) that can implement the cell spreading method (including a cell spreading step) of the invention;
Fig. 3 is a graph created based on measurement results of Experiment Example 1, the graph representing a relationship between water contact angle (a contact angle between the cell spreading substrate and water) and cell retention rate;
Fig. 4 is a graph created based on measurement results of Experiment Example 2, the graph representing a relationship between concentration of a protein (BSA) in a cell suspension and cell retention rate;
Fig. 5 is images of a pipette tip captured when a cell suspension containing BSA with a predetermined concentration was pipetted 10 times, in which white particle-shaped objects are cells adsorbing to the pipette tip; and
Fig. 6 is a graph created based on measurement results of Experiment Example 5, the graph representing a relationship between pipetting frequency and ratio of cells adherent to a pipette tip.

### Detailed Description of Embodiments

### -Cell Spreading Method-

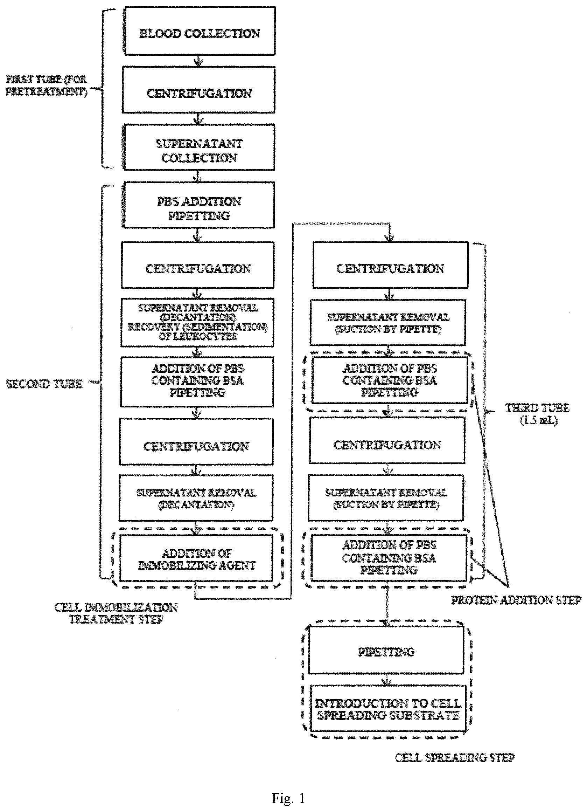

A cell spreading method of the present invention includes at least a protein addition step and a cell spreading step, and, when using a blood sample, usually includes pretreatment steps such as an anticoagulation treatment and a centrifugal treatment. The cell spreading method of the invention may further include a cell immobilization treatment step and other treatment steps as needed. Each step of such a cell spreading method can be performed, for example, in a procedure according to a flowchart depicted in Fig. 1. Hereinafter, each step included in the cell spreading method of the invention will be described in more detail.

### (Cells)

Although cells (a population) for use in the cell spreading method of the invention are not particularly limited, a typical example of the cells is those contained in a specimen collected from a human or other animals, such as blood, urine, lymph, tissue fluid, and coelomic fluid, or cultured cells (a cell line). Particularly, it is preferable to set, as target cells, rare cells such as CTCs, CECs, EPCs, nucleated erythrocytes, and other precursor cells contained in blood and the like, stem cells contained in cultured cells, specific differentiated cells, or other characteristic cells and set cells (a population) including such target cells, as cells for use in the cell spreading method.

### (Cell Suspension)

A cell suspension can be prepared, for example, by diluting a specimen likely to contain rare cells or other target cells, such as blood, urine, lymph, tissue fluid, or coelomic fluid, or a pretreated product such as a cell fraction or a purified product obtained from the specimen, with an appropriate solvent such as PBS. Additionally, the cell suspension may be one prepared by dispersing a cell line of rare cells or other target cells or a cell population containing target cells cultured for examination, research, or the like in PBS or the like. A cell suspension prepared by adding a cell line of rare cells such as CTCs as a blood cell model of a patient to a suspension of hemocytes collected from a healthy individual may be used.

In the present invention, a cell suspension that is ready for being subjected to the cell spreading step is a cell suspension that has been subjected to usually performed pretreatments (an anticoagulation treatment and a centrifugation treatment), a cell immobilization treatment step that is performed as needed, and the protein addition step,
which means a cell suspension that contains target cells such as CTCs treated with a predetermined immobilizing agent and that contains an added protein with a specific concentration that achieves both of a blocking effect against laboratory equipment and an adsorption effect on a cell spreading substrate.

### (1) Cell Immobilization Treatment Step

The cell immobilization treatment step is a step of performing an immobilization treatment of cells contained in the cell suspension, as depicted as a step performed in a second tube of Fig. 1. The immobilization treatment is a treatment that is performed to delay self-decomposition and decay of the cells to maintain form and antigenicity thereof, and can increase detectability of target cells such as CTCs.

### (Immobilizing Agent)

Examples of the immobilizing agent that is used to immobilize the cells include aldehydes such as formaldehyde and acetaldehyde; ketones such as acetone and methyl ethyl ketone; and alcohols such as ethanol and methanol. Additionally, as donors that release an immobilizing agent by being subjected to hydrolysis or the like although they themselves do not directly act as immobilizing agents, for example, formaldehyde donors are also used as one form of well-known immobilizing agents. The immobilizing agent used in the present invention is not particularly limited, but, for example, aldehydes such as formaldehyde and acetaldehyde (aldehyde group-containing compounds) are preferable.

The immobilization treatment can be performed by causing an immobilizing agent with an appropriate concentration to contact with the cells for an appropriate time. The concentration of the immobilizing agent during the immobilization treatment can be adjusted according to needs, and, for example, is approximately from 0.1 to 10 w/w%. A contact time between the immobilizing agent and the cells (an immobilization treatment time) can also be adjusted as appropriate, and, for example, is approximately from 10 minutes to one hour at room temperature.

### (Other Treatments)

Prior to the immobilization treatment of the cells, blood or a fraction prepared therefrom can be subjected to treatments other than the aforementioned immobilization treatment as needed. Particularly, when preparing a cell suspension by using a specimen collected from a human or any other animal, it is appropriate to perform necessary pretreatments in advance. Hereinafter, a description will be given of a pretreatment step for blood as a typical specimen containing CTCs. In this case, the aforementioned immobilization treatment (the cell immobilization treatment step) is generally performed for a cell suspension that has been subjected to an anticoagulation treatment and a centrifugation treatment.

### . Anticoagulation Treatment

Blood (whole blood) collected and taken out from a body coagulates over time when directly contacted with air, thereby making it impossible to recover and observe cells contained in the blood. Due to this, preferably, the collected blood is immediately subjected to an anticoagulation treatment.

Various anticoagulants for whole blood are well known and can be used according to general conditions such as concentration and treatment time. Examples of the anticoagulants include anticoagulants of a type that binds with calcium ions by chelating action to prevent coagulation by being added from a reaction system, which are typified by ethylenediamine tetraacetic acid (EDTA) and citric acid (including salts such as sodium salt), and anticoagulants of a type that prevents coagulation by forming a complex together with antithrombin III in plasma to inhibit production of thrombin, which are typified by heparin. A blood collection tube containing any of such anticoagulants in advance may be used.

### . Centrifugation Treatment

In order to obtain a cell fraction containing target cells such as rare cells from whole blood and additionally prepare a cell suspension appropriate to be subjected to the cell spreading step, centrifugation treatment is usually performed a couple of times. A technique for separating and purifying rare cells (leukocytes) such as CTCs from the whole blood by centrifugation treatment to prepare a fraction containing such cells is well known, and can be performed using an appropriate centrifuge and appropriate centrifugal conditions.

Herein, density gradient centrifugation is known as a method that can fractionate components in blood containing various kinds of cells according to specific gravity. Density gradient centrifugation is preferably used, particularly to allow only leukocytes containing target cells such as CTCs to be subjected to the cell spreading step by adding erythrocytes contained in large amount in blood.

A separation liquid that is used in density gradient centrifugation can be any as long as the separation liquid has a specific gravity appropriate to fractionate the cells in the blood and is one prepared so as to have an osmotic pressure and a pH that do not destroy the cells. For example, sucrose solutions such as commercially available FICOLL (registered trademark) and PERCOLL (registered trademark) can be used. When density gradient centrifugation is performed after adjusting so that the specific gravity of the separation liquid is smaller than the specific gravity of erythrocytes and larger than the specific gravity of leukocytes, the blood specimen can be separated into at least two layers: "a fraction containing a large amount of erythrocytes" and "a fraction containing large amounts of cells other than erythrocytes". For example, when the specific gravity of the separation liquid is set to preferably 1.113 or less, and more preferably 1.085 or less, a mixing ratio of erythrocytes into "the fraction containing large amounts of cells other than erythrocytes" can be suppressed to from 2 to 6% or therebelow. Performing cell observation by using "the fraction containing large amounts of cells other than erythrocytes" is preferable since it reduces a risk where the target cells such as CTCs are mixed into the erythrocytes and cannot be detected, thereby enabling improvement of diagnostic accuracy.

### (2) Protein Addition Step

The protein addition step is a step of adding a protein to a cell suspension, as depicted as a step performed in a third tube of Fig. 1. The protein addition step is preferably performed after the immobilization treatment step. Additionally, although the protein addition step can be performed before the immobilization treatment step, an immobilizing agent that should react with protein in the cells will also react with the added protein in the solution, and therefore it is appropriate to make long a reaction time of the immobilizing agent or make high the concentration of the immobilizing agent.

At the protein addition step, it is necessary to adjust the concentration of the protein in the cell suspension to an appropriate level so as to achieve both of prevention effect on nonspecific cell adsorption to laboratory equipment (such as the third tube and a pipette used for pipetting) and loss of the cells before being spread on the cell spreading substrate and retention effect on adsorptive capability of the cells onto a surface of the cell spreading substrate (preferably, bottom surfaces of microchambers) to enable efficient cell recovery from the cell suspension at the cell spreading step subsequent to the step. To do that, in the present invention, at the protein addition step, protein is added in such an amount that the protein in the cell suspension has a final concentration of 0.000 1% or more, and preferably 0.0005% or more, and 0.1% or less, and preferably 0.01% or less. By setting the final concentration of the protein in the cell suspension after the protein addition step to 0.1% or less, and preferably 0.01% or less, cell adsorption effect onto the cell spreading substrate is sufficiently maintained, and once the cells adsorb to the substrate, they do not come off easily even when a staining solution or the like is delivered. Additionally, by setting the final concentration of the protein in the cell suspension after the protein addition step to 0.0001% or more, and preferably 0.005% or more, suppression effect on cell adsorption to laboratory equipment is sufficiently exhibited. When the frequency of pipetting (details will be described later) before adding the cell suspension to the cell spreading substrate is set small, for example, to 10 times or less, the suppression effect on the nonspecific adsorption to laboratory equipment such as a pipette tip can be sufficiently obtained as long as the amount of the protein added is 0.0001% or more. Accordingly, while the final concentration of the protein in the cell suspension after the protein addition step is basically from 0.0001% to 0.1% in the present invention, the final concentration thereof in a preferable embodiment can be from 0.0005% to 0.01%, and, in another preferable embodiment in which the frequency of pipetting is from one to 10 times, the final concentration thereof can be from 0.0001 % to 0.01%.

As the protein that is added at the protein addition step, a protein that is generally used as a blocking agent can be used, and, for example, BSA is suitable.

The protein addition step may be repeated twice, as in the embodiment depicted in Fig. 1, repeated only once, or repeated three or more times. When repeating the protein addition step twice or more, the protein concentration in the cell suspension may be set in the above predetermined range at least at the final protein addition step immediately before moving to the cell spreading step, but, preferably, is set in the above predetermined range at all protein addition steps.

It should be noted that, before the protein addition step, for example, at a stage before the cell immobilization treatment step performed in the second tube, a protein such as BSA with a concentration in the above predetermined range or a concentration thereabove may be added as a blocking treatment agent to the cell suspension in order to suppress nonspecific cell adsorption to laboratory equipment such as a tube and a pipette tip (see Fig. 1). At such a stage, it is yet unnecessary to exactly adjust cell adsorptivity to the cell spreading substrate by the concentration of protein. Immediately before the protein addition step, centrifugation is performed to recover immobilized cells (if not immobilized, untreated cells). At that time, protein contained in the cell suspension prepared at the previous steps may be substantially all separated and removed (it is acceptable to leave a very small amount of protein unremovable even by appropriate centrifugation treatment). After that, by adding a cell dilution containing a protein with a specific concentration defined in the present invention at the protein addition step, it can be regarded that a cell suspension with a final concentration lying in the specific range defined in the invention has been prepared.

Additionally, if the amount (concentration) of protein in the cell suspension at a stage immediately before the protein addition step can be grasped, it is possible to adjust the amount (concentration) of a protein to be added at the protein addition step in consideration of the grasped amount thereof. Examples of such a protein include albumin, gamma-globulin, and other plasma proteins contained in blood (plasma), and BSA added to prevent the nonspecific adsorption before the immobilization treatment step. Additionally, when preparing a cell suspension not from blood but by using cultured cells, there may be mentioned proteins such as BSA contained in the culture medium. Furthermore, in pathological diagnosis, there may also be mentioned proteins contained in the cells of tissue pieces taken out from a patient (a human or the like). When the concentration of such a protein does not exceed any concentration in the above predetermined range, an appropriate amount of a protein can be added to the cell suspension so that the final concentration lies in the above predetermined range.

### (Laboratory Equipment)

At the cell immobilization treatment step, the protein addition step, the cell spreading step, and the like in the cell spreading method of the present invention, laboratory equipment such as a pipette tip, a micro tube is used. While there are known various materials of these pieces of laboratory equipment, the present invention preferably uses a pipette tip and/or a micro tube made of a material selected from polystylene, polypropylene, polycarbonate, polyethylene terephthalate, polymethyl methacrylate, or cycloolefin polymers, and particularly preferably made of polypropylene at least the protein addition step and/or the cell spreading step. Additionally, surface(s) of the laboratory equipment made of any of the above materials may be subjected to a treatment that further suppresses cell adhesion, like hydrophilic treatment, for example, to a treatment using a methacrylic phosphorylcholine (MPC) polymer. However, the material of the pieces of laboratory equipment is not particularly limited, and a most appropriate material can be selected in various embodiments in accordance with purposes.

On laboratory equipment made of any of the above materials, inherently, cell adsorption to equipment is relatively unlikely to occur. However, in the case of a cell suspension not containing any protein (blocking agent) with a predetermined concentration, a problem has occurred where cells adsorb nonspecifically to equipment such as a pipette tip and a micro tube on occasions such as when preparing a cell suspension containing a large number of rare cells, when continuously introducing cells to a cell spreading substrate, and when increasing pipetting frequency to evenly disperse cells and a reagent. Once cells adsorb to laboratory equipment, other cells adsorb to the cells in a chain reaction, as a result of which a large number of cells including target cells such as rare cells are left on the laboratory equipment, thus making it impossible to spread the rare cells or the like on the cell spreading substrate and recover in microchambers or the like. In the present invention, adjusting the protein in the cell suspension so as to give a predetermined final concentration enables suppression of cell adsorption to laboratory equipment even when continuously introducing the cell suspension containing a large number of rare cells by the same equipment and even when relatively increasing the frequency of pipetting.

It should be noted that, even in the cell immobilization treatment step, similarly, laboratory equipment such as a pipette tip and/or a micro tube made of polypropylene or the like can be used, but the invention is not limited thereto, and equipment in accordance with purposes can be used.

### (Pipetting)

Regarding pipetting (suspending using a pipette) that is performed at the protein addition step of the present invention, there are cases where when adding protein to a cell suspension, pipetting is performed to mix them well (referred to as first pipetting) and where when adding the cell suspension after the protein addition onto the cell spreading substrate (in other words, at the cell spreading step), pipetting is performed to eliminate uneven distribution of cells in the cell suspension so that the cells are evenly dispersed (referred to as second pipetting). In the present invention, frequencies of the first pipetting and the second pipetting can be respectively optionally set so that the cell suspension can be evenly suspended in accordance with a cell density of the prepared cell suspension and the amount of the added protein. From the viewpoint of reducing adsorption to laboratory equipment (a pipette tip) as much as possible, the frequencies of the first pipetting and the second pipetting, respectively, are preferably from one to 10 times, and more preferably from one to three times. Additionally, a total frequency of the first pipetting and the second pipetting is also preferably 10 times or less, and more preferably three times or less. When the frequency thereof is in the above range, suppression effect on the nonspecific adsorption to laboratory equipment can be sufficiently obtained even when the concentration of the protein in the cell suspension is 0.0001%.

Furthermore, a pipetting volume of pipetting at the cell spreading step can be respectively optionally set in accordance with the cell density of the prepared cell suspension and the amount of the added protein, but preferably, the pipetting is performed at a pipetting volume that is 60% or more of a volume of the cell suspension.

### (3) Cell Spreading Step

The cell spreading step is a step of spreading cells contained in the cell suspension that has been subjected to the protein addition step on the cell spreading substrate, and allowing to stand to cause the cells to adsorb onto the substrate.

### (Cell Spreading Substrate)

A cell spreading substrate typically corresponds to a flow channel substrate of a device for use in a system for cell spreading or cell observation that will be described later. By combining with a flow channel forming member to form a flow channel and introducing a cell suspension to the flow channel, the cell spreading substrate is used in such a manner as to spread cells in the cell suspension on a surface of the cell spreading substrate (the flow channel substrate). However, the cell spreading substrate by which the cell spreading method of the present invention can be performed is not limited to such an embodiment, and can be any cell spreading substrate as long as it enables a cell suspension to be spread on the surface thereof and then enables cells contained in the cell suspension to adsorb thereonto. For example, the cell spreading substrate may be a microscope slide or a bottom surface of a cell culture dish.

### (Contact Angle between Water and Substrate)

The cell spreading substrate used in the present invention has a contact angle with water of 60 degrees or more, and preferably from 70 to 90 degrees. When the contact angle is in such a range, adding the cell suspension on the cell spreading substrate and allowing it to stand enables the cells in the cell suspension to adsorb to the substrate with sufficient adsorption strength. The contact angle between water and the cell spreading substrate in the invention is assumed as a static contact angle measured by a curve fitting technique.

### •Cell Solidification Method: Material and Structure of Cell Spreading Substrate

In order to improve efficiency in detecting target cells such as rare cells, it is necessary to solidify the cells, i.e., to fix positions of the cells on the cell spreading substrate. Solidifying the cells facilitates specification of the positions of cells targeted for observation, and also enables recovery of the detected target cells as needed.

A technique for solidifying cells can be roughly classified into a technique in which a movable range of the cells is limited by a surface structure of the cell spreading substrate (a structural technique) and a technique in which cells are caused to adsorb and immobilized by a physical interaction that occurs due to surface properties of the cell spreading substrate (an interaction technique). The present invention uses at least the interaction technique that causes cells to adsorb onto the cell spreading substrate, and preferably additionally uses the structural technique in combination therewith.

In the present invention, as the interaction technique for solidification, the contact angle of the cell spreading substrate with water is adjusted to the specific range (60 degrees or more, and preferably from 70 to 90 degrees) as mentioned above. To do that, the cell spreading substrate may be produced by using a material having a contact angle with water in such a range, or the cell spreading substrate may be surface-modified so as to have a contact angle with water in such a range. For example, it is preferable to use a cell spreading substrate made of polystylene or polycarbonate that is relatively highly hydrophobic and thus facilitates adsorption of cells. Additionally, in the case of using such a hydrophobic cell spreading substrate made of plastic, when considering provision of a flow channel on an upper part of the cell spreading substrate to introduce a cell suspension, preferably, provision of a microchamber-like structure that will be described next on the cell spreading substrate to accommodate cells, and the like, the cell spreading substrate may be appropriately hydrophilized in a range that satisfies the above conditions of the contact angle by a UV/ozone treatment that applies UV in an air atmosphere or an oxygen plasma treatment in order to provide an affinity to the cell suspension.

On the other hand, as the structural technique for solidification, an example may be mentioned where a plurality of minute chambers (microchambers) or grooves is formed on the surface of the cell spreading substrate, and the movement of cells is limited only to within the microchambers or the grooves. When the structural technique is not used, the surface of the cell spreading substrate may be made smooth.

Although the shape of the microchambers is not particularly limited, preferred is, for example, an inverted truncated conical shape with a flat bottom surface and a tapered side surface. A diameter of the bottom surfaces of the microchambers and a depth of the microchambers can be adjusted as appropriate so that cells in an amount appropriate to observe can be recovered and accommodated. For example, preferably, the diameter of the bottom surfaces ranges from 20 to 500 µm and the depth of the microchambers ranges from 20 to 500 µm so as to be able to accommodate one to 100 cells per microchamber. It should be noted that diameters of various cells (excluding erythrocytes) in blood are generally said to be from 5 to 100 µm, and diameters of rare cells such as CTCs are said to be approximately from 10 to 100 µm.

Although arrangement of the plurality of microchambers on the surface of the cell spreading substrate (flow channel substrate) is not particularly limited, it is preferable to adjust directions of arrays and intervals between the microchambers so as to increase a cell recovery rate (a rate of cells that were able to be recovered in the microchambers from among all the cells in the suspension) as much as possible. For example, the microchambers are preferably arrayed in such a manner that cells settle out in the microchambers at at least some one place between a flow inlet and a flow outlet when the cell suspension is delivered to the flow channel.

In the present invention, particularly, it is suitable to spread cells on a cell spreading substrate made of polystylene and having microchambers, which the substrate has been subjected to hydrophilic treatment as needed, and has a contact angle with water of 60 degrees or more (preferably from 70 to 90 degrees). Causing the cells to adsorb to the microchambers with bottom surfaces made of polystylene enables the cells to be efficiently separated and recovered from the cell suspension, and also hardly causes loss of the cells separated and recovered once even when the flow of the cell suspension or any other liquid on the cell spreading substrate (in the flow channel) is relatively strong.

### •Blocking Treatment

In the present invention, at the protein addition step, a protein with a specific concentration is added to the cell suspension, and the contact angle of the cell spreading substrate with water is set in the specific range, whereby, even without performing any blocking treatment on a region except for opening portions of the microchambers on the cell spreading substrate, it is possible to suppress cells from adsorbing to such a region while the cell suspension is flowing (by contrast, spreading the cell suspension and then allowing it to stand enables the cells to adsorb to the bottom surfaces of the microchambers accommodating the cells). However, if necessary, as is done conventionally (see PTL 1: WO 2014/007191), the region except for the opening portions of the microchambers on the cell spreading substrate may be subjected to a blocking treatment in advance. Performing a blocking treatment on such a region in advance can further reduce the possibility of cell adhesion and can ensure that rare cells or the like are recovered in the microchambers and caused to adsorb to the bottom surfaces thereof.

### •Cell Spreading Device

A cell spreading device is constructed by the flow channel substrate and the flow channel forming member, in which a space closed by them serves as a flow channel that can be filled with a liquid such as a cell suspension delivered. Near terminal ends of an upstream side and a downstream side of the flow channel are formed the flow inlet and the flow outlet for causing the above various kinds of liquids to flow in and out. The flow channel substrate and the flow channel forming member may be adapted to be attachable to and detachable from each other by a means of engagement, screw fixation, sticking, or the like, from the viewpoint of easy observation and easy maintenance.

Using a cell spreading substrate as described above as the flow channel substrate of the cell spreading device enables production of a cell spreading device suitable for performing the cell spreading method of the present invention. The cell spreading substrate as the flow channel substrate in the present embodiment is adapted to enable use of at least the interaction technique that allows for cell adsorption, as described above, and preferably is adapted to enable additional use of the structural technique in combination therewith.

The flow channel forming member may be constructed by a frame member that forms sidewalls of the flow channel, the frame member creating a void to provide a predetermined height to the flow channel and forming a two-dimensional range of the flow channel, and a top plate member that is mounted on the frame member to form a ceiling of the flow channel. Near the terminal ends of the upstream side and the downstream side of the flow channel, the flow channel forming member (a lid member) forming the ceiling of the flow channel is provided with opening portions corresponding to the flow inlet and the flow outlet of the flow channel. The top plate member may include a space (a reservoir) that communicates with either the flow inlet or the flow outlet, the space temporarily storing a liquid such as a cell suspension.

Similarly to the cell spreading substrate (the flow channel substrate), the flow channel forming member can be made of, for example, polystylene, and, in that case, may be subjected to a blocking treatment as needed, similarly to the region except for the opening portions of the microchambers on the cell spreading substrate.

The height of the flow channel (an interval between the flow channel substrate and the top plate member, namely, a thickness of the frame member) is preferably from 50 to 500 µm, and more preferably from 50 to 100 µm. When the height of the flow channel is in such a range, rare cells in the cell suspension in the flow channel can be easily moved by a force of liquid delivery, and clogging due to the cells in the flow channel hardly occurs, so that the cells can be smoothly spread.

At the cell spreading step, the cell suspension may be delivered onto the cell spreading substrate (the flow channel substrate of the cell spreading device) and allowed to stand for a predetermined time, for example, for one minute or more to cause cells to settle out. When providing microchambers on the surface of the cell spreading substrate and accommodating the cells therein to recover, an amount of flow (a flow rate) and a direction of the delivered liquid may be changed in order to increase the cell recovery efficiency. For example, by setting to a pattern where after performing liquid delivery for a short time, the cell suspension is allowed to stand for a short time (an intermittent liquid delivery) or a pattern where after performing liquid delivery in a forward direction from the flow inlet to the flow outlet, liquid delivery is performed in a direction opposite thereto, it is possible to minimize target cells such as rare cells left in the region except for the microchambers on the flow channel substrate or discarded without being recovered in the microchambers until the end.

### (Use of Cell Spreading Method)

Use of the cell spreading method of the present invention is not particularly limited, and the cell spreading method of the invention can be used in various embodiments in accordance with purposes. Typically, the cell spreading method of the invention is used to detect target cells such as rare cells, in which cells are spread on the cell spreading substrate, followed by (fluorescent) staining and observation of the cells, and the like. When performing such an embodiment by using the cell spreading device as described above, cell suspension delivery for spreading cells is performed, and then, sequentially, delivery of a (fluorescent) staining solution, delivery of a washing solution, and the like are performed. In the invention, the protein concentration in the cell suspension is adjusted in the specific range to maintain cell adsorptivity to the cell spreading substrate, so that the number of cells desorbed from and washed away from the surface of the cell spreading substrate (the inside of the microchambers) when additionally delivering a (fluorescent) staining solution, a washing solution, and other liquid(s) after the delivery of the cell suspension, can be reduced, for example, to 20% or less of cells recovered from the cell suspension at the cell spreading step (conversely, a cell retention rate of 80% or more can be obtained).

As a staining solution for specifying the presence of cells when observing or detecting the cells, an aqueous solution of a nuclei staining agent can be used. Nuclei staining agents are fluorescent dye molecules that emit fluorescence by intercalation into double-stranded DNA. Examples of such nuclei staining agents include Hoechst-based dyes (such as Hoechst 33342 and Hoechst 33258) that have cell membrane permeability to allow nuclei staining of living cells and DAPI (4', 6-diamidino-2-phenylindole) that have no cell membrane permeability and therefore cannot stain nuclei of living cells, but can stain nuclei of dead cells permeable due to altered cell membranes.

Additionally, as a staining solution for observing or detecting a specific kind of cell, an aqueous solution of a complex of an antibody to a protein specific to the cell (a so-called cell marker) and fluorescent dye molecules can be used. For example, as proteins that are for identifying CTCs from among leukocytes recovered by using the cell spreading substrate and that are expressed in epithelial cellular CTCs and hardly expressed in leukocytes, there may be mentioned EpCAM (Epithelial Cell Adhesion Molecule) expressed on cell surfaces and cytokeratin (CK) expressed in cells, both of which are known as cancer cell markers. On the other hand, examples of proteins that are expressed in leukocytes and hardly expressed in CTCs include CD45 expressed on cell surfaces and known as a leukocyte marker. Accordingly, a staining solution corresponding to a specific kind of a targeted cell can be prepared by using a staining agent directly labelling any such cell marker, for example, a complex of an antibody to the cell marker and fluorescent dye molecules, a staining agent indirectly labelling any such cell marker, for example, a combination of an antibody to the cell marker (a first antibody) and a complex of an antibody to the first antibody (a second antibody) and fluorescent dye molecules, or the like.

### <Means (Apparatus-System) for Performing Cell Spreading Method>

Means for performing the cell spreading method of the present invention are not particularly limited, and the protein addition step, the cell spreading step, and, as needed, other steps such as the cell immobilization treatment step may be performed in order by using well-known means.

In a preferable embodiment, in the cell spreading method of the present invention, at least the cell spreading step can be performed by using an apparatus-system provided with a liquid delivery system mechanism for spreading a cell suspension that has been subjected to the protein addition step on the cell spreading substrate (the flow channel substrate of the cell spreading device). Furthermore, when performing (fluorescent) staining and observation of cells, and the like following the cell spreading step in order to detect target cells such as rare cells, as described above, those can be performed by using a cell detection apparatus-system that can perform them all together. Additionally, such an apparatus-system for cell spreading or cell detection can also be linked with an apparatus-system for performing the cell immobilization treatment step and the protein addition step.

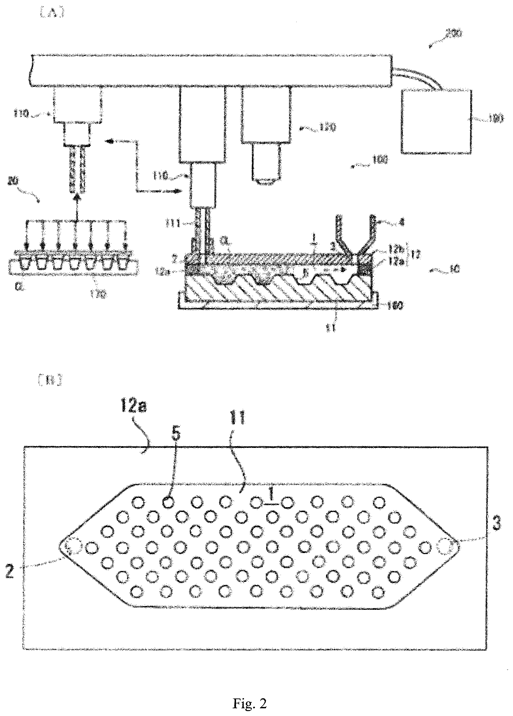

Fig. 2 illustrates one embodiment of a cell detection system (200). The cell detection system (200) includes a cell detection apparatus (100), a cell spreading device (100), a reagent container (20), and a control means (190) for controlling various kinds of devices included in the cell detection apparatus. The cell detection apparatus (100) includes a liquid delivery system mechanism (110) for delivering various kinds of liquids to a flow channel (1) of the cell spreading device (10), an optical system mechanism (120) for observing cells (CL) recovered by the cell spreading device (100), a cell spreading device holder (160) for holding the cell spreading device (100), and a reagent container holder (170) for holding the reagent container (20). The liquid delivery system mechanism (110) and the optical system mechanism (120) are desirably provided with spatial movement means for enabling suction and discharge of liquid and cell observation at an optional position. The optical system mechanism (120) can be configured in accordance with a microscope, and preferably is configured in accordance with a fluorescent microscope so that, particularly, cells stained with a fluorescent dye can be observed. Preferably, the control means (190) can control the liquid delivery system mechanism (110) by a program to enable a predetermined liquid in an predetermined amount to be introduced onto the cell spreading substrate (11) (into the flow channel (5)) at a predetermined flow rate in a predetermined timing so that particularly the cell spreading step can be automatically performed.

### •Liquid Delivery System Mechanism

The liquid delivery system mechanism is a mechanism that moves between respective portions for storing a cell suspension, a (fluorescent) staining solution, a washing solution, and other reagents in the reagent container and the flow inlet of the cell recovery device to perform suction and discharge of the liquids. Specifically, the liquid delivery system mechanism suctions a predetermined amount of a liquid such as the cell suspension contained in the reagent container, discharges the liquid in the flow inlet of the cell recovery device at a predetermined rate, and introduces into the flow channel. Additionally, after finishing the predetermined treatment by liquid delivery, the mechanism suctions and drains the liquid filling the flow channel from the flow inlet, and discharges in a waste liquid storing portion of the reagent container. The liquid delivery system mechanism can be constructed by using, for example, a syringe pump, a replaceable tip, an actuator movable in an X-axis direction (a lateral direction in the drawing) and a Z-axis direction (a vertical direction in the drawing), or the like. The syringe pump has an ability that enables suction and discharge of a cell suspension, a washing solution, and the like at a desired flow rate at the cell spreading step and other steps. In the present invention, the "replaceable tip" included in the liquid delivery system mechanism corresponds to one of "pieces of laboratory equipment" on which nonspecific adsorption of cells should be suppressed.

### . Reagent Container

The reagent container accommodates various kinds of liquids that need to be delivered to the flow channel in performing the cell spreading step and other steps for cell observation, such as a cell suspension, staining solutions (such as a nuclei staining solution and an immunofluorescent staining solution), and a washing solution. For example, liquids having relatively high preservability, such as a washing solution, can be accommodated in a sealed state into predetermined portions of the reagent container in advance, and liquids that need to be prepared immediately before cell observation, such as a cell suspension and a staining solution, are adapted so as to be accommodatable by adding into predetermined portions of the reagent container after preparation. As the cell suspension, the present invention uses a cell suspension that has been subjected to the immobilization treatment step and the protein addition step. Solutions that are used a plurality of times in cell observation, such as a staining solution and a washing solution, may be accommodated at volumes corresponding to respective steps in separate places in advance, or when repeatedly using a solution having the same composition at the respective steps, a total dose of the solution therefor may be accommodated in one place in advance. Additionally, as needed, the reagent container is adapted to be provided with a portion for storing a waste liquid suctioned and drained from the flow channel after liquid delivery. In the invention, the "reagent container" (the portion in which a cell suspension is accommodated) corresponds to one of the "pieces of laboratory equipment" on which the nonspecific adsorption of cells should be suppressed.

### -Cell Spreading Kit-

A cell spreading kit that can be used to perform the cell spreading method of the present invention includes, for example, a cell dilution containing a protein with a concentration of from 0.0001% to 0.1%, and preferably a protein with a concentration of from 0.0005% to 0.01%, as described above, or the protein and a solvent for preparing the cell dilution, and a cell spreading substrate having a contact angle with water of 60 degrees or more, and preferably from 70 to 90 degrees.

Such a cell spreading kit may include other reagents, equipment for preparing the reagents, a manual, and the like as needed. As the other regents, for example, there may be mentioned a nuclei staining solution and a washing solution, as described above, or a nuclei staining agent and a washing agent for preparing them, and a dilution (a solvent such as PBS) therefor.

The cell spreading method of the present invention includes a step of adding to a cell suspension a protein in such an amount as to give a final concentration of from 0.0001 % to 0.1% (a protein addition step) and a step of spreading cells contained in the cell suspension that has been subjected to the protein addition step on a cell spreading substrate having a contact angle with water of 60 degrees or more, and allowing to stand to cause the cells to adsorb onto the cell spreading substrate (a cell spreading step).

The cell spreading method of the invention may include an embodiment in which, at the protein addition step, the protein is added in such an amount as to give a final concentration of from 0.0005% to 0.01%.

The cell spreading method of the invention may include an embodiment in which, at the protein addition step, the protein is added in such an amount as to give a final concentration of from 0.0001% to 0.01%, and pipetting at the cell spreading step is performed from one to 10 times.

The cell spreading method of the invention may include an embodiment in which the pipetting at the cell spreading step is performed from one to three times.

The cell spreading method of the invention may include an embodiment in which the contact angle with water of the cell spreading substrate is from 70 to 90 degrees.

The cell spreading method of the invention may include an embodiment in which the cells contained in the cell suspension are cells that have been subjected to a cell immobilization treatment.

The cell spreading method of the invention may include an embodiment in which laboratory equipment that is used at the protein addition step and/or used for pipetting at the cell spreading step is made of polypropylene.

The cell spreading method of the invention may include an embodiment in which laboratory equipment that is used at the protein addition step and/or used for pipetting at the cell spreading step is coated with a methacryl phosphorylcholine (MPC) polymer or bovine serum albumin (BSA).

The cell spreading method of the invention may include an embodiment in which the pipetting at the cell spreading step is performed at a pipetting volume that is 60% or more of a volume of the cell suspension.

A cell spreading kit of the present invention may include (a) a cell dilution containing a protein with a concentration of from 0.0001% to 0.1% or the protein and a solvent for preparing the cell dilution and (b) a cell spreading substrate having a contact angle with water of 60 degrees or more.

The cell spreading kit of the invention may include, as the (a), a cell dilution containing the protein with a concentration of from 0.0005% to 0.01% or the protein and a solvent for preparing the cell dilution.

The cell spreading substrate (b) used in the cell spreading kit of the invention may have a contact angle with water of from 70 to 90 degrees.

### Examples

### [Experimental Example 1] (Screening of Contact Angle between Water and Substrate that Provides Effect on Cell Adsorption to Substrate)

### [1-1] Preparation of Cell Suspension

A culture solution of CCRF-CEM cells (a human T-lymphocytic leukemia-derived cell line) was centrifuged at 200 G for four minutes, and the separated cells were washed with PBS twice. The cells were suspended in PBS to prepare 200 µl of a cell suspension with a concentration of 3.0 × 10⁵/ml. The cell suspension was subjected to a cell immobilization treatment by adding formaldehyde so as to give a final concentration of 0.4% in a PROTEOSAVE SS 1.5 ml Microtube (Product No. MS-4215M, registered trademark, Sumitomo Bakelite, ultra-hydrophilized, and made of polypropylene) at room temperature. The obtained cell suspension was used as the sample of Experimental Example 1.

### [1-2] Production of Cell Spreading Device

A cell spreading substrate molded using a predetermined mold was prepared, which substrate was made of polystylene and provided with microchambers. The substrate and a flow channel forming member (a flow channel lid) were irradiated with UV/O₃ for 20 seconds to make them hydrophilic, and then assembled together into a cell spreading device. The cell spreading substrate was not subjected to a blocking treatment with BSA. The microchambers had a diameter of 100 µm, a depth of 50 µm, and an inverted conical shape with a flat bottom. A pitch representing a distance between centers of the adjacent microchambers was 200 µm, and voidage (voids are regions where opening portions of the microchambers are not present in a direction (namely, a flow channel transverse direction) perpendicular to a flow channel longitudinal direction (namely, a downstream direction from the flow inlet to the flow outlet), and the voidage is a ratio of a void length to a transverse length of the substrate) was 0%. Additionally, the flow channel had a height of 100 µm, a width (transverse direction) of 15 mm, and a length (longitudinal direction) of 40 mm In other words, the flow channel has a cross-sectional area of 1.5 mm² and a volume of 60 mm³ (= 0.06 mL).

Regarding the cell spreading substrate produced as described above, contact angles with water before and after the hydrophilic treatment performed by irradiating with UV/O₃ for 20 seconds were measured by a dynamic contact angle meter ("FTA125" manufactured by FTA Co.), and found to be 100 degrees and 80 degrees, respectively. Substrates having various contact angles with water were produced in the same manner as above except that the UV/O₃ irradiation time was changed.

### [1-3] Adsorption of Cells to Cell Spreading Substrate

[1-3-1] First, a tube and a 50 mL Terumo syringe were connected to the flow channel of each cell spreading device produced in [1-2], and ultrapure water was introduced into the flow channel to pre-wet (flow rate: 40 mL/min, volume: 40 mL), whereby the flow channel was filled with the ultrapure water.
[1-3-2] Next, a tube and a 1 mL Hamilton syringe were connected to the flow channel, and PBS was introduced (flow rate: 0.1 mL/min, volume: 150 µL) to fill the flow channel with the PBS.
[1-3-3] Subsequently, using the same Hamilton syringe, the cell suspension prepared in [1-1] was introduced into the flow channel (flow rate: 0.1 mL/min, volume: 150 µL) and allowed to stand for 1 minute. After that, a first image capturing was performed by an inverted microscope.
[1-3-4] Furthermore, using the same Hamilton syringe, PBS was introduced into the flow channel (flow rate: 1 mL/min, volume: 500 µL) to wash an inside of the flow channel. After that, in the same field of view as the first image capturing, a second image capturing was performed by an inverted microscope.

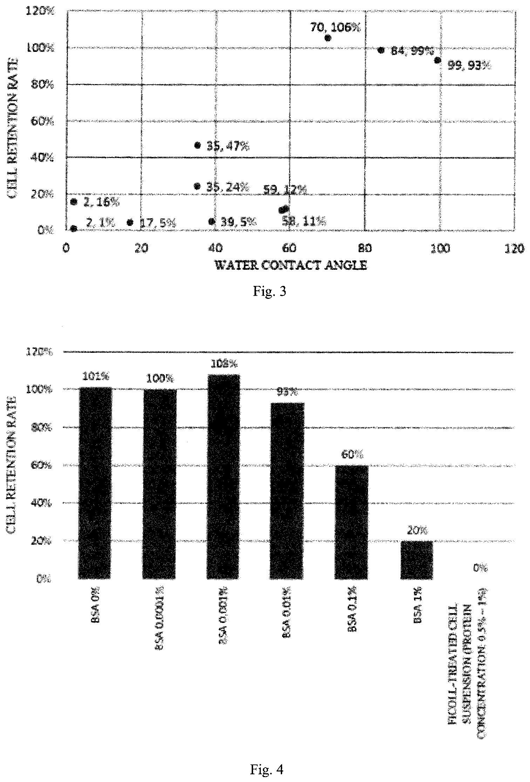

In each of the first captured image (before introducing the PBS for washing) and the second captured image (after introducing the PBS for washing), the number of cells present in the microchambers were counted to calculate a ratio of the latter to the former (a cell retention rate). Fig. 3 shows results. The results of the screening indicated that adsorption force of the cells to the cell spreading substrate greatly changed at a boundary at which the contact angle between water and the substrate was 60 degrees, and when the contact angle was 60 degrees or more the cell retention rate was 80% or more.

### [Experimental Example 2] (Protein Concentration that Provides Effect on Cell Adsorption to Substrate)

A cell suspension was prepared in the same manner as [1-1] of Experimental Example 1, and then a protein (BSA) was added to give a final concentration of 0%, 0.0001%, 0.001%, 0.01%, 0.1% or 1%. Additionally, as a control, whole blood after four days from collection in a blood collection tube CYTO-CHEX (registered trademark) was treated with Ficoll (GE Co.) to remove erythrocytes, and then washed with PBS once to produce a specimen (containing, besides leukocytes, protein in an amount of approximately from 0.5% to 1%). These five cell suspensions were used as samples of Experimental Example 2.

As a cell spreading substrate, a substrate hydrophilized by irradiating with UV/O₃ for 30 seconds and having a contact angle with water of 80 degrees was used, which the substrate was made of polystylene and provided with microchambers. Using each of the cell suspension samples of Experimental Example 2 and the cell spreading substrate, cell retention rate was calculated in the same procedure as in [1-3] of Experimental Example 1. Fig. 4 shows results. When the BSA concentration was 1%, adsorptivity of cells to the substrate has been almost lost (cell retention rate: 20%), and even at the BSA concentration of 0.1%, the cell adsorptivity has been significantly reduced (cell retention rate: 60%). By contrast, when the BSA concentration was 0.01% or less, the cell adsorptivity has been sufficiently maintained (cell retention rate: 93% or more), which indicates that a staining solution and a washing solution can be delivered at a relatively large flow rate.

### [Experimental Example 3] (Cell Adsorption Effect onto Substrate and Adaptability to Staining Protocol)

There were prepared an MDA-MB 231 cell suspension and a leukocyte suspension subjected to immobilization treatment (treatment time: three days; room temperature) in the same manner as [1-1] of Experimental Example 1 except for using MDA-MB231 cells (a human breast cancer-derived cell line) and leukocytes instead of CCRF-CEM cells. These cell suspensions were mixed together to prepare 100 µL of a cell suspension containing 3 × 10⁵ immobilized MDA-MB231 cells and 1000 immobilized leukocytes, and furthermore, a protein (BSA) was added to give a final concentration of 0.01%. Additionally, as a control, a cell suspension was prepared in the same procedure as above except for adding the protein (BSA) to give a final concentration of 1%. These two cell suspensions were used as samples of Experimental Example 3.

As a cell spreading substrate, a substrate hydrophilized by irradiating with UV/O₃ for 30 seconds and having a contact angle with water of 80 degrees was used, which substrate was made of polystylene and provided with microchambers. Additionally, as staining solutions, anti-CK staining solution, anti-CD45 staining solution, and cell nuclei staining solution (Hoechst solution) were used. The anti-CK staining solution is a solution of PE (phycoerythrin)-labeled anti-CK monoclonal antibody (clone name: CAM5.2, Becton, Dickinson and Company), and the anti-CD45 staining solution is a solution of APC (allophycocyanin)-labeled anti-CD45 monoclonal antibody (clone name J33, Beckman Coulter, Inc). MDA-MB231 cells (epithelial cells) can be immunostained with anti-CK staining solution, and leukocytes can be immunostained with anti-CD45 staining solution. In other words, in reacting with each of the aforementioned staining solutions, if a detected cell is CD45-negative, CK-positive, and stained with Hoechst 33342 solution, the cell can be determined to be an MDA-MB231 cell, and if a detected cell is CD45-positive, CK-negative, and stained with Hoechst 33342 solution, the cell can be determined to be a leukocyte.

Using each of the cell suspension samples and the cell spreading substrate of Experimental Example 3, first, ultrapure water, PBS, and each cell suspension were introduced into the flow channel in the same procedure as in [1-3-1] to [1-3-3] of Experimental Example 1 (provided that the first image capturing of [1-3-3] was not performed).

Furthermore, using the same Hamilton syringe as in [1-3-3], a mixed solution of the anti-CK staining solution, the anti-CD45 staining solution, and the cell nuclei staining solution (Hoechst 33342 solution) was introduced (flow rate: 0.1 mL/min, volume: 150 µL) into the flow channel to stain cells collected in the microchambers.

After that, fluorescent images corresponding to each of PE, APC, and Hoechst 33342 were captured to identify MDA-MB231 cells and count the number thereof. Then, a ratio of the number of the MDA-MB231 cells collected in the cell spreading substrate to the number (3 × 10⁵) of the MDA-MB231 cells in the initial cell suspension (a cell collection rate) was calculated.

The experiment was performed three times in each of cases where the final concentrations of the protein (BSA) were 0.01% and 1%. As a result, when the final concentration of the protein was 0.01%, average cell collection rate was 100% (SD 10%), whereby the cell suspension containing the protein with the concentration specified in the present invention can be considered to have high detection ability, so that adaptability to the staining protocol was confirmed. On the other hand, at the final concentration of the protein of 1%, the average cell collection rate was 10% (SD 10%), whereby significantly reduced detection ability was confirmed.

### [Experimental Example 4] (Effect on Suppression of Cell Adsorption to Laboratory Equipment made of Polypropylene)

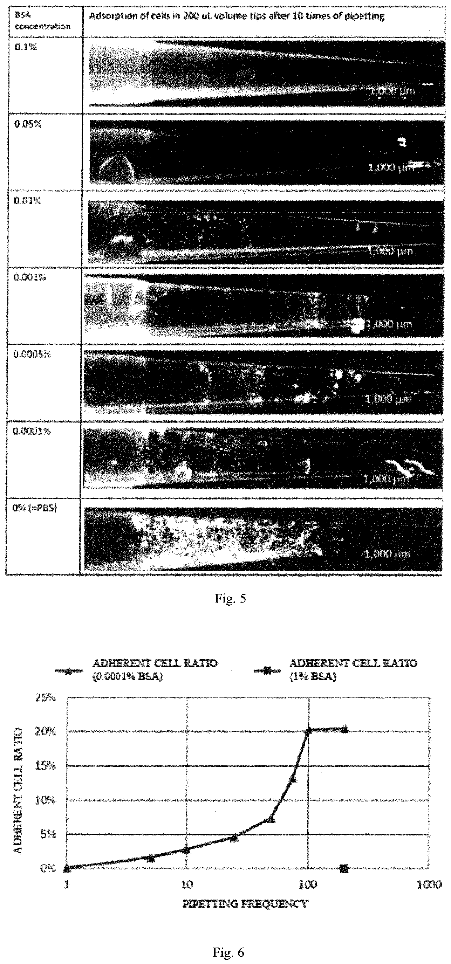

Immobilization treatment of leukocytes was performed in the same procedure as in [1-1] of Experimental Example 1, and immediately after that, 1 µl of cell nuclei staining solution (Hoechst 33342 solution) was added to the obtained cell suspension. The cell concentration of the cell suspension was adjusted to 3.5 × 10⁶/ml by using PBS, and 100 µl of the cell suspension was added in a "PROTEOSAVE (registered trademark) SS 1.5 ml Microtube" (Sumitomo Bakelite Co. Ltd.), and centrifuged at 300 G for one minute to remove the supernatant. PBS solutions (BSA/PBS) with BSA concentrations (corresponding to the final concentrations of the protein in cell suspensions to be prepared) of 0%, 0.0001%, 0.001%, 0.01%, 0.05%, and 0.1% were added in an amount of 100 µl to the cell fraction after the centrifugation, and the samples were pipetted 10 times at a pipetting volume being 68 µl by using an ART 200 µl pipette tip (registered trademark, molecular Bio Products; made of polypropylene) and a 200 µl PIPETMAN. Furthermore, in order to wash off extra liquid droplets adherent to the pipette tip, PBS was pipetted 10 times at the same pipetting volume. After that, the pipette tip was photographed by a fluorescent microscope to evaluate an effect on suppression of cell adhesion to the pipette tip. Fig. 5 shows results. In cases where BSA with the final concentration of 0.0001% or more was added, the amount of adherent cells was obviously small compared to the case where no BSA was added, indicating the effect on suppression of cell adhesion to the laboratory equipment.

The results of Experimental Examples 2 to 4 hereinabove are shown together in the following Table. From these results, in the present invention, the amount of the protein to be added to the cell suspension is basically set to a range of amounts such that the final concentration is from 0.0001 % to 0.1%, and preferably can be set to a range of amounts such that the final concentration is from 0.0005% to 0.01%. Additionally, Experimental Example 5 shows that when pipetting frequency is limited (to 10 times or less), the amount of the protein can be preferably set to the range of amounts such that the final concentration is from 0.0001 % to 0.01%.

**Table 1**

| BSA concentration in cell suspension | Adsorption effect onto base (Experimental Example 2) | Suppression effect on adhesion to base material (Experimental Example 3) | Suppression effect on adhesion to base material when pipetting frequency is limited (Experimental Example 4) |
|---|---|---|---|
| 0% | ⊚ | × | Δ |
| 0.0001% | ⊚ | Δ | ○ |
| 0.0005% | ⊚ | ○ | ○ |
| 0.001% | ⊚ | ○ | ○ |
| 0.01% | ○ | ○ | ⊚ |
| 0.05% | - | ⊚ | ⊚ |
| 0.1% | Δ | ⊚ | ⊚ |
| 1% | × | ⊚ | ⊚ |

### [Experimental Example 5] (Effect on Suppression of Cell Adhesion to Laboratory Equipment made of Polypropylene)

To the cell fraction after the centrifugation prepared in Experimental Example 4 was added 100 µl of a PBS solution (BSA/PBS) with a BSA concentration (corresponding to the final concentration of the protein in a cell suspension to be prepared) of 0.0001%, and the samples were pipetted 1, 5, 10, 25, 50, 75, 100, and 200 times at a pipetting volume being 68 µl by using an ART 200 µl pipette tip (registered trademark, molecular Bio Products; made of polypropylene) and a 200 µl PIPETMAN. Furthermore, in order to wash off extra liquid droplets adherent to the pipette tip, PBS was pipetted 10 times at the same pipetting volume . After that, the pipette tip was photographed by a fluorescent microscope to count the number of cells adherent to the pipette tip. The ratio of the number of cells adherent to the pipette tip to the number of cells in 100 µL was defined as an adherent cell ratio, and the ratio was plotted every pipetting frequency. Fig. 6 shows a created graph. In the case where the final concentration was 0.0001%, reducing the pipetting frequency to 10 times or less was found to be effective in enabling the adherent cell ratio to be reduced to 5% or less. Reference Signs List

1: Flow channel
2: Flow inlet
3: Flow outlet
4: Reservoir
5: Microchamber
10: Cell spreading device
11: Flow channel substrate (cell spreading substrate)
12: Flow channel forming member
12a: Frame member
12b: Top plate member
20: Reagent container
CL: Cell suspension
100: Cell detection apparatus
110: Liquid delivery system mechanism
111: Tip
120: Optical system mechanism
160: Cell spreading device holder
170: Reagent container holder
190: Control means
200: Cell detection system

## Claims

1. A cell spreading method comprising:
a step of adding to a cell suspension a protein in such an amount as to give a final concentration of from 0.0001 % to 0.1% (a protein addition step); and
a step of spreading cells contained in the cell suspension that has been subjected to the protein addition step on a cell spreading substrate having a contact angle with water of 60 degrees or more, and allowing to stand to cause the cells to adsorb onto the cell spreading substrate (a cell spreading step).

2. The cell spreading method according to claim 1, wherein, at the protein addition step, the protein is added in such an amount as to give a final concentration of from 0.0005% to 0.01%.

3. The cell spreading method according to claim 1, wherein, at the protein addition step, the protein is added in such an amount as to give a final concentration of from 0.0001% to 0.01%, and pipetting at the cell spreading step is performed from one to 10 times.

4. The cell spreading method according to claim 3, wherein the pipetting at the cell spreading step is performed from one to three times.

5. The cell spreading method according to claim 1, wherein the contact angle with water of the cell spreading substrate is from 70 to 90 degrees.

6. The cell spreading method according to claim 1, wherein the cells contained in the cell suspension are cells that have been subjected to a cell immobilization treatment.

7. The cell spreading method according to claim 1, wherein laboratory equipment that is used at the protein addition step and/or used for pipetting at the cell spreading step is made of polypropylene.

8. The cell spreading method according to claim 1, wherein laboratory equipment that is used at the protein addition step and/or used for pipetting at the cell spreading step is coated with a methacryl phosphorylcholine (MPC) polymer or bovine serum albumin (BSA).

9. The cell spreading method according to claim 3, wherein the pipetting at the cell spreading step is performed at a pipetting volume that is 60% or more of a volume of the cell suspension.

10. A cell spreading kit comprising:
(a) a cell dilution containing a protein with a concentration of from 0.0001 to 0.1% or the protein and a solvent for preparing the cell dilution; and
(b) a cell spreading substrate having a contact angle with water of 60 degrees or more.

11. The cell spreading kit according to claim 10 comprising, as the (a), a cell dilution containing the protein with a concentration of from 0.0005% to 0.01% or the protein and a solvent for preparing the cell dilution.

12. The cell spreading kit according to claim 10, wherein the contact angle with water of the cell spreading substrate (b) is from 70 to 90 degrees.
